# EUROPÄISCHE PATENTANMELDUNG

(11) **EP 2 135 543 A1**
(43) Veröffentlichungstag der Anmeldung: **23.12.2009**
(21) Anmeldenummer: 09162097.1
(22) Anmeldetag: 05.06.2009
(51) Int. Cl.: A61B 1/005, A61M 25/00, A61M 25/01

(54) **Vorrichtung zum minimalinvasiven Einführen in ein physiologisches Lumen**

(30) Priorität: 17.06.2008 DE 102008002479
(71) Anmelder: Biotronik VI Patent AG, 6341 Baar (CH)
(72) Erfinder: Winter, Alexander, 18057 Rostock (DE); Bakczewitz, Frank, 18055 Rostock (DE)
(74) Vertreter: Lindner-Vogt, Karin L.

(57) **Zusammenfassung**

Eine Vorrichtung zum minimalinvasiven Einführen in ein Lumen eines Hohlorgans umfasst eine schlauchförmige Wand (3) mit einer Vielzahl in diese eingebetteter piezoelektrischer Fasern (4).

## Beschreibung

Der Erfindung betrifft eine Vorrichtung zum minimalinvasiven Einführen in ein physiologisches Lumen. Die Erfindung betrifft weiterhin ein Verfahren zur Herstellung einer derartigen Vorrichtung.

Grundsätzlich werden solche Vorrichtungen in der Regel als Katheter realisiert. Herkömmliche Katheterschäfte bestehen üblicherweise aus einem Mehrschichtverbund aus Polymer- und/oder Metalltuben, die koaxial ineinander liegen. Je nach Anwendung wird eine optimal erscheinende Materialkombination ausgewählt. Anschließend besteht keine Möglichkeit mehr, Einfluss auf die Mechanik oder die Geometrie des Katheters zu nehmen. Aus der US 5,415,633 und der US 6,083,170 sind Katheter bekannt, welche an ihrem distalen Ende zur Verformung desselben ein Steuerelement aufweisen.

Es ist daher Aufgabe der vorliegenden Erfindung, einen Katheter mit verbesserten mechanischen Eigenschaften zu schaffen.

Diese Aufgabe wird durch die Merkmale des Anspruchs 1 gelöst. Der Kern der Erfindung besteht darin, in der Wand eines Katheters mindestens eine Faser anzuordnen, deren Länge gezielt mittels einer Steuer-Einrichtung reguliert werden kann. Hierdurch lassen sich sowohl die mechanischen als auch die geometrischen Eigenschaften des Katheters gezielt und kontrolliert an die jeweiligen Bedürfnisse anpassen.

Weitere vorteilhafte Ausführungen der Erfindung ergeben sich aus den Unteransprüchen. Merkmale und Einzelheiten der Erfindung ergeben sich aus der nachfolgenden Beschreibung eines Ausführungsbeispiels anhand der Zeichnungen.

Es zeigen:
- Fig. 1: eine perspektivische Schnitt-Darstellung durch ein Ausführungsbeispiel der Erfindung,
- Fig. 2: eine weitere schematische Darstellung des Ausführungsbeispiels gemäß Fig. 1 und
- Fig. 3: eine schematische Schnitt-Darstellung durch die Wand eines erfindungsgemäßen Katheters im Bereich der längenverstellbaren Fasern.

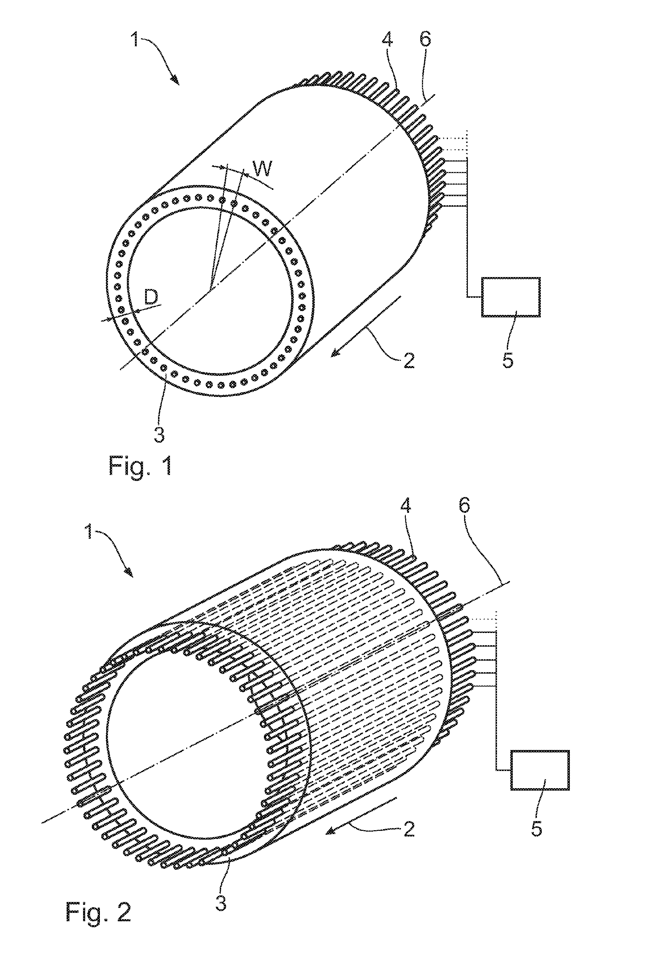

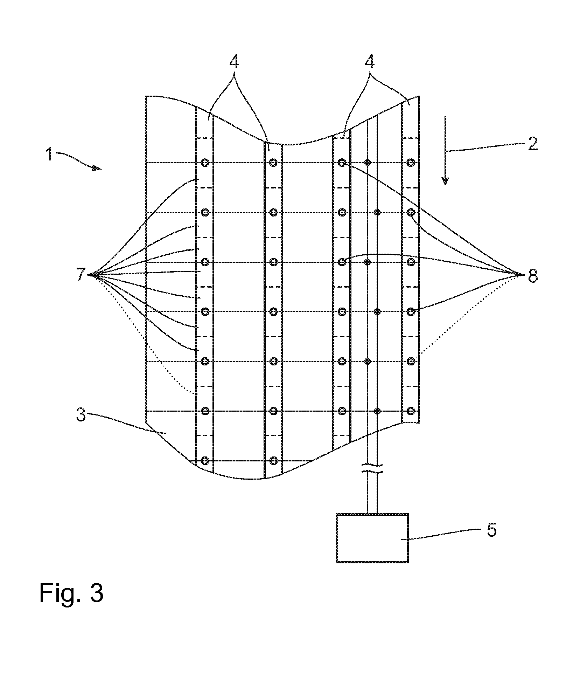

Im Folgenden wird unter Bezugnahme auf die Fig. 1 bis 3 ein Ausführungsbeispiel der Erfindung beschrieben. Die Vorrichtung umfasst einen schlauchförmigen Hohlkörper 1, welcher sich zumindest abschnittsweise entlang einer Längsrichtung 2 erstreckt. Der Hohlkörper 1 ist insbesondere hohlzylindrisch ausgebildet und umfasst eine Wand 3 mit einer Dicke D. Die Dicke D liegt im Bereich von 100 µm bis 1 mm. Die Wand 3 ist aus einem Verbundwerkstoff, insbesondere einem Faserverbundwerkstoff. Sie ist zumindest teilweise aus einem polymeren Kunststoff, welcher eine bettende Matrix bildet. Die Wand 3 kann insbesondere mehrschichtig ausgebildet sein.

Die Wand 3 ist flexibel, vorzugsweise elastisch verformbar und hat im Ausgangszustand einen kreisringförmigen Querschnitt. Andere Querschnitte sind jedoch ebenfalls denkbar. In die Polymermatrix der Wand 3 ist eine Vielzahl piezoelektrischer Fasern 4 eingebettet. Die piezoelektrischen Fasern 4 dienen als Beispiel für faserförmige Elemente, deren Länge mittels einer in den Fig. 1 und 2 nur schematisch dargestellten Steuer-Einrichtung 5 kontrolliert veränderbar ist. Die Steuer-Einrichtung 5 ist Teil der erfindungsgemäßen Vorrichtung. Hierdurch sind die geometrischen und/oder mechanischen Eigenschaften der Wand 3 mittels der Steuer-Einrichtung 5 gezielt veränderbar. Die piezoelektrischen Fasern 4 sind beispielsweise aus Blei-Zirkonat-Titanat.

Gemäß der in den Figuren dargestellten Ausführungsform ist eine Vielzahl parallel zueinander ausgerichteter, piezoelektrischer Fasern 4 in der Wand 3 vorgesehen. Die Fasern 4 sind parallel zur Längsrichtung 2 ausgerichtet und erstrecken sich in Längsrichtung 2 über die gesamte Länge der Wand 3. Die piezoelektrischen Fasern 4 sind vollständig in die Polymermatrix der Wand 3 eingebettet. Vorzugsweise sind die piezoelektrischen Fasern 4 gleichmäßig über den Umfang der Wand 3 verteilt. Auch eine ungleichmäßige Verteilung z. B. in vier Paketen über den Umfang könnte vorteilhaft sein. Die piezoelektrischen Fasern 4 sind in einem Winkelabstand W bezüglich einer Mittelachse 6 des Hohlkörpers 1 zueinander angeordnet. Hierbei liegt der Winkelabstand W im Bereich von 0,1° bis 10°.

Eine Anordnung der piezoelektrischen Fasern 4 konzentrisch zur Mittelachse 6 alternativ und/oder zusätzlich zu den piezoelektrischen Fasern 4 in Längsrichtung 2 ist selbstverständlich ebenso möglich und kann je nach Anwendung besonders vorteilhaft sein. Entsprechend ist in einer alternativen, in den Figuren nicht dargestellten Ausführungsform vorgesehen, die piezoelektrischen Fasern 4 spiralförmig um die Mittelachse 6 anzuordnen.

Die Anzahl der piezoelektrischen Fasern 4 liegt im Bereich von 1 bis 1000, insbesondere im Bereich von 10 bis 100, insbesondere im Bereich von 20 bis 50.

Die piezoelektrischen Fasern 4 weisen einen Durchmesser im Bereich von 5 µm bis 500 µm, insbesondere im Bereich von 100 µm bis 300 µm auf.

Die piezoelektrischen Fasern 4 dienen als piezoelektrische Aktoren, welche von der Steuer-Einrichtung 5 einzeln und/oder gruppenweise ansteuerbar sind. Hierzu ist die Steuer-Einrichtung 5 elektrisch leitend mit jeder der piezoelektrischen Fasern 4 verbunden. Durch Anlegen einer elektrischen Spannung an die piezoelektrischen Fasern 4 mittels der Steuer-Einrichtung 5 ist die Länge der piezoelektrischen Fasern 4 kontrolliert veränderbar. Hierdurch sind die geometrischen und/oder mechanischen Eigenschaften der Wand 3 mittels der Steuer-Einrichtung 5 gezielt veränderbar.

Die piezoelektrischen Fasern 4 können in Längsrichtung 2 in eine Vielzahl von Abschnitten 7 unterteilt sein, wobei an jeden einzelnen Abschnitt 7 unabhängig von den anderen mittels der Steuer-Einrichtung 5 eine Spannung mit einstellbarer Polarität und Amplitude anlegbar ist. Hierzu weist jede piezoelektrische Faser 4 eine Vielzahl von elektrischen Verbindungsstellen 8 mit der Steuer-Einrichtung 5 auf.

Im Folgenden wird die Funktionsweise der Vorrichtung beschrieben. Die Vorrichtung, welche insbesondere als Katheter zum minimal invasiven Einführen in ein Lumen eines Hohlorgans dient, ist im Ausgangszustand, in welchem keine elektrische Spannung an den piezoelektrischen Fasern 4 anliegt, flexibel, elastisch und zumindest abschnittsweise entlang der Längsrichtung 2 ausgerichtet. Der Katheter ist über seine gesamte Länge geradlinig, d. h. entlang der Längsrichtung 2 ausgerichtet. Wird jedoch an mindestens eine der piezoelektrischen Fasern 4 eine elektrische Spannung einer bestimmten Polarität angelegt, ändert diese Faser 4 ihre Länge, wird beispielsweise länger. Dies kann zur Änderung der Geometrie des Katheters genutzt werden. Insbesondere lässt sich durch Änderung der Länge der piezoelektrischen Fasern 4 in einem bestimmten Winkelbereich bezüglich der Mittelachse 6, welcher insbesondere kleiner als 180° ist, gezielt eine Krümmung des Katheters herbeiführen. Eine Verlängerung der Fasern 4 führt hierbei zu einer konvexen Oberfläche der Wand 3 in dem besagten Winkelbereich bezüglich der Mittelachse 6, während eine Verkürzung der Fasern 4 zu einer konkaven bzw. sattelförmigen Oberfläche in diesem Winkelbereich führt. Dieser Effekt lässt sich durch Anlegen einer Spannung mit umgekehrter Polarität an die piezoelektrischen Fasern 4, welche in einem dem ersten Winkelabschnitt bezüglich der Mittelachse 6 gegenüberliegenden zweiten Winkelabschnitt angeordnet sind, verstärken.

Entsprechend lassen sich die Flexibilität und/oder Elastizität bzw. allgemein die mechanischen Eigenschaften des Katheters durch Anlegen einer Spannung gleicher Polarität und Amplitude an jeweils bezüglich der Mittelachse 6 einander gegenüberliegende piezoelektrische Fasern 4 verändern. Insbesondere ist es möglich, den Katheter durch Anlegen der gleichen Spannung an alle piezoelektrischen Fasern in einem bestimmten Abschnitt bezüglich der Längsrichtung 2 zu versteifen.

Bei Anordnung zumindest einiger Fasern 4 konzentrisch zur Mittelachse 6 lässt sich darüber hinaus der Durchmesser bzw. Umfang des Katheters durch Anlegen einer Spannung an diese Fasern 4 vorzugsweise auch dynamisch verändern.

Im Folgenden wird die Herstellung der erfindungsgemäßen Vorrichtung beschrieben. Zunächst wird die Wand 3 des schlauchförmigen Hohlkörpers 1 in einem Extrusionsverfahren hergestellt. Hierbei werden die piezoelektrischen Fasern 4 koextruiert, d. h. gleichzeitig mit der Extrusion der Wand 3 in diese eingebettet. Dies führt zu einer besonders zuverlässigen Verbindung zwischen den piezoelektrischen Fasern 4 und der Wand 3 des Katheters. Anschließend wird die Steuer-Einrichtung 5 mit den piezoelektrischen Fasern 4 verbunden. Hierzu ist insbesondere ein Laserlöt-, Lasermikroschweiß- oder ein sogenanntes Laser Droplet Welding-Verfahren vorgesehen.

## Patentansprüche

1. Vorrichtung zum minimalinvasiven Einführen in ein physiologisches Lumen umfassend
a. einen sich zumindest abschnittsweise entlang einer Längsrichtung (2) erstreckenden Hohlkörper (1) mit einer Wand (3) und
b. eine Steuer-Einrichtung (5),
c. wobei die Wand (3) mindestens ein faserförmiges Element (4) aufweist, dessen Länge mittels der Steuer-Einrichtung (5) kontrolliert veränderbar ist.

2. Vorrichtung gemäß Anspruch 1, **dadurch gekennzeichnet, dass** das mindestens eine faserförmige Element (4) in eine Polymermatrix eingebettet ist.

3. Vorrichtung gemäß einem der vorhergehenden Ansprüche, **dadurch gekennzeichnet, dass** sich das mindestens eine faserförmige Element (4) über die gesamte Länge der Wand (3) erstreckt.

4. Vorrichtung gemäß einem der vorhergehenden Ansprüche, **dadurch gekennzeichnet, dass** eine Vielzahl parallel zueinander ausgerichteter, faserförmiger Elemente (4) vorgesehen ist.

5. Vorrichtung gemäß einem der vorhergehenden Ansprüche, **dadurch gekennzeichnet, dass** die geometrischen und/oder mechanischen Eigenschaften der Wand (3) mittels der Steuer-Einrichtung (5) veränderbar sind.

6. Vorrichtung gemäß einem der vorhergehenden Ansprüche, **dadurch gekennzeichnet, dass** mindestens ein faserförmiges Element (4) parallel zur Längsrichtung (2) ausgerichtet ist.

7. Vorrichtung gemäß einem der vorhergehenden Ansprüche, **dadurch gekennzeichnet, dass** mindestens ein faserförmiges Element (4) konzentrisch zum Umfang der Wand (3) des Hohlkörpers (1) angeordnet ist.

8. Vorrichtung gemäß einem der vorhergehenden Ansprüche, **dadurch gekennzeichnet, dass** mindestens ein faserförmiges Element (4) spiralförmig um eine Mittelachse (6) in das Material der Wand (3) eingebettet ist.

9. Vorrichtung gemäß einem der vorhergehenden Ansprüche, **dadurch gekennzeichnet, dass** das mindestens ein faserförmiges Element (4) vollständig in das Material der Wand (3) eingebettet ist.

10. Vorrichtung gemäß einem der vorhergehenden Ansprüche, **dadurch gekennzeichnet, dass** es sich bei den faserförmigen Elementen (4) um piezoelektrische Aktoren handelt.

11. Vorrichtung gemäß Anspruch 4, **dadurch gekennzeichnet, dass** die Länge der faserförmigen Elemente (4) mittels der Steuer-Einrichtung (5) einzeln und/oder gruppenweise kontrolliert, insbesondere abschnittsweise veränderbar ist.

12. Verfahren zur Herstellung einer Vorrichtung gemäß einem der vorhergehenden Ansprüche umfassend die folgenden Schritte:
a. Extrusion einer Wand (3) eines Hohlkörpers (1),
b. gleichzeitiges Einbetten mindestens eines faserförmigen, piezoelektrischen Elements (4) in die Wand (3) mittels eines Coextrusions-Verfahrens.
